# EUROPÄISCHE PATENTANMELDUNG

(11) **EP 1 757 299 A1**
(43) Veröffentlichungstag der Anmeldung: **28.02.2007**
(21) Anmeldenummer: 05107790.7
(22) Anmeldetag: 25.08.2005
(51) Int. Cl.: A61K 33/26, A61K 31/715, A61P 7/06

(54) **Eisen(III)-Komplexverbindungen zur Behandlung von Eisenmangel-Zuständen bei Patienten mit chronisch-entzündlicher Darmerkrankung**

(71) Anmelder: VIFOR (INTERNATIONAL) AG, 9001 St. Gallen (CH)
(72) Erfinder: Die Erfindernennung liegt noch nicht vor
(74) Vertreter: Gille Hrabal Struck Neidlein Prop Roos

(57) **Zusammenfassung**

Es wird die Verwendung von Eisen(III)-Komplexverbindungen mit Kohlenhydraten oder Derivaten davon zur Herstellung eines Arzneimittels zur Behandlung von Eisenmangel-Zuständen bei Patienten mit chronisch entzündlicher Darmerkrankung offenbart, insbesondere Morbus Crohn und Colitis ulcerosa.

## Beschreibung

Die vorliegenden Erfindung betrifft neue therapeutische Anwendungen von Eisen(III)-Komplexverbindungen mit Kohlehydraten oder Derivaten davon, insbesondere mit Dextrinen oder Oxidationsprodukten von Dextrinen, nämlich zur Herstellung von Arzneimitteln zur Behandlung von Eisenmangel-Zuständen bei Patienten mit chronisch entzündlichen Darmerkrankungen, insbesondere Morbus Crohn und/oder Colitis ulcerosa.

Eisenmangel ist der häufigste Spurenelementmangel weltweit. Ca. 2 Milliarden Menschen weltweit leiden an Eisenmangel oder EisenmangelAnämie (E,M, DeMoeyer, "Preventing and controlling iron deficiency anaemia through primary health care", World Health Organization, Genf, 1989, ISBN 92 4 154249 7),

Aus der WO 95/351 13 ist die Verwendung von Eisen(III)-oxid als Wirkstoff zur Behandlung von Immunschwächerkrankungen, insbesondere AIDS, bekannt.

Aus der DE 1467980 sind therapeutisch verwendbare Eiseninjektionspräparate und Verfahren zu Ihrer Herstellung bekannt,

Aus der US 3076798 sind Verfahren zur Herstellung von Eisen(III)-Polymaltose-Komplexverbindungen bekannt, die zur parenteralen Verabreichung geeignet sind.

Aus der WO 04/037865 ist die Verwendung von Eisen-Kohlenhydrat-Komplexen zur Behandlung oder Prophylaxe von Eisenmangelzuständen bekannt.

Aus der WO 03/087164 sind Eisen-Komplexverbindungen mit hydrierten Dextrinen zur Behandlung oder Prophylaxe von Eisenmangelzuständen bekannt.

Aus der WO 02/46241 sind Eisen(III)-Pullulan-Komplexverbindungen und ihre Verwendung zur Behandlung oder Prophylaxe von Eisenmangelzuständen bekannt.

WO 99/48533 offenbart Eisen-Dextran-Verbindungen zur Behandlung von Eisenmangelanämie, die hydriertes Dextran mit einem bestimmten Molekulargewicht von ca, 1000 Dalton umfassen.

Eisensulfat ist dafür bekannt, dass es relativ häufig unangenehme dosisabhängige Nebenreaktionen, wie gastrointestinale Störungen oder eine Verfärbung der Zähne hervorruft. Eisen aus Eisensalz-Verbindungen unterliegt der passiven Diffusion freier Eisenionen. Das Eisen kann in den Kreislauf eintreten und dadurch Nebenreaktionen oder eine Eisenvergiftung hervorrufen. Dementsprechend ist auch der LD50-Wert bei weißen Mäusen mit 230 mg Eisen/kg relativ niedrig.

In Oski et al, "Effect of Iron Therapy on Behavior Performance in Nonannemic, Iron-Deficient Infants", PEDIATRICS 1983; Band 71 ; 877-880 ist die Verwendung von Eisen-Dextran offenbart. Die parenterale Verwendung von Eisen-Dextran ist nachteilig, weil ein Dextran-induzierter anaphylaktischer Schock auftreten kann.

Entzündliche Darmerkrankungen (inflammatory bowel disease, IBD) umfassen eine Gruppe von Erkrankungen des Gastrointestinaltrakts, die durch intestinale Entzündung und einen chronisch Verlauf mit ständigen Rückfällen gekennzeichnet sind. IBD wurde traditionell entweder als Colitis ulcerosa oder Morbus Crohn charakterisiert, basierend auf klinischen, radiologischen, endoskopischen und histologischen Kriterien. Obwohl die Ätiologie von IBD noch der Definition bedarf, legen neuere klinische und experimentelle Studien nahe, dass der Auslöser und die Pathogenese dieser Erkrankungen multifaktoriell sind und dass Wechselwirkungen zwischen genetischen, Umwelt- und Immunfaktoren involviert sind.

Entzündliche Darmerkrankungen sind weltweit nicht gleichmäßig verbreitet. Es besteht eine klare Tendenz zu einem vermehrten Auftreten in entwickelten Ländern verglichen mit weniger entwickelten Ländern. Das Vorkommen von IBD in Europa beträgt ca. 390 Fälle pro 100.000 Personen. Eine Extrapolation dieser Zahlen auf die europäische Population von ca. 580 Millionen ergibt eine geschätzte Zahl von 2,2 Millionen Personen, die von IBD betroffen sind (Loftus EV, Jr., Gastroenterolgy 2004, 126, 11504-1517). Colitis ulcerosa und Morbus Crohn werden am häufigsten bei älteren Heranwachsenden und jungen Erwachsenen diagnostiziert, können aber in jedem Lebensalter auftreten.

Colitis ulcerosa ist eine Schleimhauterkrankung, die üblicherweise das Rektum befällt und sich dann in die benachbarten Bereiche ausdehnt, so dass das Kolon ganz oder teilweise befallen wird. Die Ausbreitung erfolgt kontinuierlich, ohne dass Bereiche nicht betroffener Schleimhaut verbleiben. Die Hauptsymptome von Colitis ulcerosa sind heftiger Durchfall, rektale Blutungen, Schleimabgang und krampfartige Bauchschmerzen. Die Schwere der Symptome korreliert mit der Ausdehnung der Erkrankung.

Morbus Crohn kann jeden Bereich des Gastrointestinaltrakts vom Mund bis zum Anus befallen, betrifft aber am häufigsten den Dünndarm und/oder das Kolon. Die Entzündung ist transmural und segmental, wobei normale Bereiche zwischen Bereichen erkrankten Darms vorhanden sind. Folgen der Entzündung schließen Fistelbildung an anderen Darmschlingen, Harnblase, Vagina oder Perianalhaut, abdominale oder perianale Abszesse und intestinale Verengungen ein. Die Lokalisierung und der Verlauf der Erkrankung beeinflussen die klinischen Manifestationen. Die häufigsten Symptome sind Durchfall, krampfartige Bauchschmerzen, Fieber, Anorexie und Gewichtsverlust.

Extraintestinale Manifestationen von Colitis ulcerosa und Morbus Crohn können multiple Organsysteme betreffen, wie Augen, Haut und Gelenke, genauso wie gastrointestinale Organe einschließlich Leber und Gallenblase,

Die Behandlung umfasst die Gabe antientzündlicher Mittel, u,U, von Antibiotika, und eine Ernährungsumstellung. Gelegentlich kann eine Operation erforderlich sein. Weiter erfolgt häufig eine Psychotherapie, einerseits zur Bewältigung von Stress, der mit als Auslöser gilt, andererseits zur Behandlung von Depressionen, die häufig als Folge der chronisch immer wiederkehrenden Beschwerden auftreten (s. z.B. Pschyrembel, Klinisches Wörterbuch, 256. Auflage, de Gruyter, S. 302/303, S. 443; http://familydoctor.org oder http://www.mayoclinic.com).

Eisenmangel tritt bei Patienten mit chronisch entzündlicher Darmerkrankung häufig als Komplikation auf. Chronische intestinale Blutungen können dazu führen, dass mehr Eisen verloren geht, als durch die Nahrung aufgenommen wird. Übliche orale Eisenpräparate, im allgemeinen Eisen(II)-Salze, verursachen häufig schwere gastrointestinale Nebenwirkungen, was zu einer schlechten Patienten-Copmpliance führt. Die orale Eisentherapie kann die Läsionen des intestinalen Gewebes durch die Katalyse der Bildung von reaktiven Sauerstoffspezies verstärken. Da freies Eisen ein starker Katalysator des Bildung von reaktiven Sauerstoffspezies ist, kann die orale Eisen(II)-Therapie für Patienten mit chronisch entzündlicher Darmerkrankung sogar schädlich sein. Orale Eisen(II)-Präparate werden schlecht absorbiert und führen zu hohen faecalen Eisenkonzentrationen, und ein signifikanter Anteil des faecalen Eisens ist für die katalytische Aktivität verfügbar. Wenn Eisen in Kontakt mit der entzündeten intestinalen Mucosa kommt, kann es die Produktion reaktiver Sauerstoffspezies erhöhen und dadurch Gewebeschädigungen verstärken. Daher ist es für Patienten mit chronischer entzündlicher Darmerkrankung besonders wichtig, gut verträgliche Eisenpräparate zur Verfügung zu haben.

Eisen(III)-Polymaltose-Komplex enthält Eisen in nicht-ionischer Form, die weniger toxisch ist. Es treten bei Gabe von Verbindungen dieses Typs weniger Nebenwirkungen auf, und die Patienten-Compliance ist gegenüber Eisen(II)-sulfat verbessert (Jacobs, P., Wood, L., Bird, AR., Hematol, 2000, 5:77-83). Es gibt jedoch noch keine Erfahrungen oder Berichte über die Anwendung von Eisen(III)-Polymaltose-Komplex bei Patienten mit chronischentzündlicher Darmerkrankung.

Die Erfinder stellten sich daher die Aufgabe, gut verträgliche Eisenverbindungen zu finden, die geeignet sind, die Eisenmangel-Zustände bei Patienten mit chronisch entzündlicher Darmerkrankung zu behandeln.

In einer Studie konnten sie nachweisen, dass Eisen(III)-Komplexverbindungen mit Kohlehydraten, insbesondere mit Polymaltose (Maltodextrin) besonders verträglich sind und eine hohe Patienten-Compliance besitzen. Überraschend war dabei, dass unter der Behandlung mit den Eisen(III)-Komplexen kein oxidativer Stress auftrat, im Gegensatz zur Behandlung mit Eisen(II)-sulfat, unter der eine signifikante Erhöhung von Plasma-Malondialdehyd (MDA), einem Marker der Lipid-Peroxidation, beobachtet wurde.

Oxidativer Stress, insbesondere die Lipidperoxidation, wird mit einem erhöhten Risiko, an Herzinfarkt, Krebs und Atherosklerose zu erkranken, in Verbindung gebracht. Die oxidative Modifizierung von Low-Density Lipoprotein (LDL) wird für die Atherogenese verantwortlich gemacht (s, in Tuomainen et al., Nutrition Research, Vol 19, No.8, pp. 1121-1132, 1999 angegebene Referenzen).

Eisen(III)-Polymaltose-Komplex-Verbindungen führen zwar nur zu einer langsamen Erhöhung des Ferritinspiegels, werden aber effizienter für die Hämoglobin-Synthese verwendet (T.-P. Tuomainen et al., aaO., p. 1 127). Auf der Basis dieses Ergebnisses stellten die Erfinder die vorliegende Erfindung fertig.

Gegenstand der Erfindung ist daher die Verwendung von Eisen(III)-Komplexverbindungen mit Kohlehydraten oder Derivaten davon zur Herstellung eines Arzneimittels zur Behandlung von Eisenmangel-Zuständen bei Patienten mit chronisch entzündlicher Darmerkrankung.

Unter Eisenmangel-Zustand gemäß der Erfindung wird ein Zustand verstanden, bei dem Hämoglobin, Eisen und Ferritin im Plasma vermindert sind und Transferrin erhöht ist, was zu einer erniedrigten Transferrin-Sättigung führt.

Der erfindungsgemäß zu behandelnde Zustand umfasst Eisenmangelanämie und Eisenmangel ohne Anämie, Die Einteilung kann beispielsweise durch den Hämoglobinwert und den Wert für die Transferrinsättigung (%) erfolgen. Referenzwerte für Hämoglobin, bestimmt durch Durchflusszytometrie oder die photometrische Cyanhämoglobinmethode, und Referenzwerte für Eisen, Ferritin und Transferrin sind beispielsweise gelistet in der Referenzdatenbank der Charité, Institut für Laboratoriumsmedizin und Pathobiochemie (http://www.charite.de/ilp/routine/parameter.html) und in Thomas, L. Labor und Diagnose, TH Book Verlagsgesellschaft, Frankfurt/Main 1998, Die Transferrinsättigung ist bei Patienten ohne Eisenmangel in der Regel >16 %. Die Normalwerte sind in der später folgenden Tabelle III angegeben.

Laut M. Wick, W. Pinggera, P. Lehmann, Eisenstoffwechsel - Diagnostik und Therapien der Anämien, 4., erw. Aufl. Springer Verlag Wien 1998 lassen sich alle Formen des Eisenmangels klinisch-chemisch nachweisen. Dabei geht im allgemeinen eine erniedrigte Ferritin-Konzentration mit kompensatorisch erhöhtem Transferrin und niedriger Transferrinsättigung einher.

Unter chronisch entzündlicher Darmerkrankung (inflammatory bowel disease, IBD) wird eine chronische Entzündung des Verdauungstrakts verstanden, insbesondere Morbus Crohn und Colitis ulcerosa.

Erfindungsgemäß anwendbare Eisen(III)-Komplexverbindungen mit Kohlehydraten schließen bevorzugt diejenigen ein, worin Kohlenhydrate aus der Gruppe ausgewählt werden, die aus Dextranen und Derivaten davon, Dextrinen und Derivaten davon sowie Pullulan, Oligomeren und/oder Derivaten davon besteht. Die genannten Derivate umfassen insbesondere die hydrierten Derivate. Besonders bevorzugt sind Eisen(III)-Komplexverbindungen mit Dextrinen oder Oxidationsprodukten davon. Beispiele der Herstellung der erfindungsgemäßen Eisen(III)-Komplexverbindungen finden sich beispielsweise in den eingangs erwähnten Patentschriften DE 14679800, WO 04037865 Al, US 3076798, WO 03/087164 sowie WO 02/46241, deren Offenbarungsgehalt insbesondere hinsichtlich der Herstellverfahren hier vollumfänglich eingeschlossen sein soll. Der Begriff der erfindungsgemäß bevorzugt verwendeten "Dextrine" ist eine Sammelbezeichnung für verschiedene niedere und höhere Polymere aus D-Glucose-Einheiten, die bei unvollständiger Hydrolyse von Stärke entstehen. Dextrine können ferner durch Polymerisation von Zuckern hergestellt werden (z.B. WO 02083739 A2, US 20030044513 A1, US 3766165). Zu den Dextrinen gehören die Maltodextrine bzw. Polymaltosen, die durch enzymatische Spaltung von zum Beispiel Mais- oder Kartoffelstärke mit alpha-Amylase hergestellt werden und die durch den Hydrolysegrad ausgedrückt durch den DE-Wert (Dextrose-Äquivalent) charakterisiert werden. Polymaltose kann erfindungsgemäß auch durch saure Hydrolyse von Stärken, insbesondere von Dextrinen erhalten werden. Die Herstellung der erfindungsgemäß anwendbaren Eisen(III)-Komplexverbindungen erfolgt im allgemeinen durch Umsetzung von Eisen(II)- oder (III)-salzen, insbesondere Eisen(III)-chlorid, mit den Dextrinen, insbesondere Polymaltose, oder Oxidationsprodukten der Dextrine in wässriger alkalischer Lösung (pH > 7) und anschließender Aufarbeitung. Die Herstellung gelingt auch im schwach sauren pH-Bereich. Bevorzugt sind jedoch alkalische pH-Werte von beispielsweise > 10.

Das Anheben des pH-Wertes erfolgt bevorzugt langsam bzw. allmählich, was beispielsweise dadurch erfolgen kann, dass zunächst eine schwache Base zugesetzt wird, beispielsweise bis zu einem pH von etwa 3; anschließend kann dann mit einer stärkeren Base weiter neutralisiert werden. Als schwache Base kommen beispielsweise Alkali-oder Erdalkalicarbonate, - bicarbonate, wie Natrium- und Kaliumcarbonat oder -bicarbonat oder Ammoniak infrage. Starke Basen sind beispielsweise Alkali- oder Erdalkalihydroxide, wie Natrium-, Kalium-, Calcium-oder Magnesiumhydroxid.

Die Umsetzung kann durch Erwärmen begünstigt werden, Beispielsweise können Temperaturen in der Größenordnung von 15°C bis zur Siedetemperatur angewendet werden. Es ist bevorzugt, die Temperatur allmählich zu steigern. So kann beispielsweise zunächst auf etwa 15 bis 70°C erwärmt und allmählich bis zum Sieden gesteigert werden.

Die Reaktionszeiten liegen beispielsweise in der Größenordnung von 15 Minuten bis zu mehreren Stunden, z.B. 20 Minuten bis 4 Stunden, beispielsweise bei 25 bis 70 Minuten, z. B. 30 bis 60 Minuten.

Nach erfolgter Umsetzung kann die erhaltene Lösung beispielsweise auf Raumtemperatur abgekühlt und gegebenenfalls verdünnt und gegebenenfalls filtriert werden. Nach dem Abkühlen kann der pH-Wert durch Zugabe von Säure oder Base auf den Neutralpunkt oder leicht darunter, beispielsweise auf Werte von 5 bis 7 eingestellt werden. Als Basen können beispielsweise die vorstehend zur Umsetzung genannten verwendet werden. Säuren schließen beispielsweise Salzsäure und Schwefelsäure ein. Die erhaltenen Lösungen werden gereinigt und können direkt zur Herstellung von Arzneimitteln verwendet werden. Es ist aber auch möglich, die Eisen(III)-Komplexe aus der Lösung zu isolieren, beispielsweise durch Ausfällen mit einem Alkohol, wie einem Alkanol, beispielsweise Ethanol. Die Isolierung kann auch durch Sprühtrocknung erfolgen, Die Reinigung kann in üblicher Weise erfolgen, insbesondere zur Entfernung von Salzen, Dies kann z. B. durch Umkehrosmose erfolgen, wobei eine derartige Umkehrosmose z. B. vor der Sprühtrocknung oder vor dem direkten Einsatz in Arzneimitteln durchgeführt werden kann.

Die erhaltenen Eisen(III)-Komplexe weisen beispielsweise einen Eisengehalt von 1 0 bis 40 % Gew./Gew., insbesondere 20 bis 35% Gew./Gew. auf. Sie sind im allgemeinen gut wasserlöslich. Man kann daraus neutrale wässrige Lösungen mit beispielsweise 1 % Gew./Vol. bis 20 % Gew./Vol. Eisengehalt herstellen. Diese Lösungen lassen sich thermisch sterilisieren.

Bezüglich der Herstellung von Eisen(III)-Polymaltose-Komplexverbindungen kann auch auf die US 3076798 verwiesen werden.

In einer bevorzugten Ausführungsform der Erfindung wird eine Eisen(III)-hydroxid-Polymaltose-Komplexverbindung verwendet. Bevorzugt besitzt diese die Eisen(III)-Polymaltose-Komplexverbindung ein Molekulargewicht im Bereich von 20000 bis 500000, in einer bevorzugten Ausführungsform 30000 bis 80000 Dalton (bestimmt mittels Gelpermeationschromatographie, beispielsweise wie von Geisser et al. In Arzneim. Forsch/Drug Res. 42(11), 12,1439-1452 (1992), Absatz 2.2. 5. beschrieben). Eine besonders bevorzugte Eisen(III)-hydroxid-Polymaltose-Komplexverbindung ist das im Handel erhältlich Maltofer® der Firma Vifor AG, Schweiz, In einer weiteren bevorzugten Ausführungsform wird eine Eisen(III)-Komplexverbindung mit einem Oxidationsprodukt von einem oder mehreren Maltodextrinen verwendet. Diese ist beispielweise erhältlich aus einer wässrigen Eisen(III)-Salzlösung und einer wässrigen Lösung des Produktes der Oxidation von einem oder mehreren Maltodextrinen mit einer wässrigen Hypochloritlösung bei einem pH-Wert im alkalischen Bereich, wobei beim Einsatz von einem Maltodextrin dessen Dextrose-Äquivalent bei 5 bis 37 und beim Einsatz eines Gemisches aus mehreren Maltodextrinen das Dextrose-Äquivalent des Gemisches bei 5 bis 37 und das Dextrose Äquivalent der am Gemisch beteiligten einzelnen Maltodextrine bei 2 bis 40 liegt. Das gewichtsmittlere Molekulargewicht Mw der so erhaltenen Komplexe beträgt beispielsweise 30 kDa bis 500 kDa, bevorzugt 80 bis 350 kDa, besonders bevorzugt bis zu 300 kDa (bestimmt mittels Gelpermeationschromatographie, beispielsweise wie von Geisser et al. In Arzneim. Forsch/Drug Res. 42(11), 12,1439-1452 (1992), Absatz 2.2. 5. beschrieben). Diesbezüglich kann beispielsweise auf die WO 2004037865 A1 verwiesen werden, deren Offenbarungsgehalt vollumfänglich in vorliegender Anmeldung eingeschlossen sein soll,

Bezüglich der Herstellung von Eisen-Komplexverbindungen mit hydrierten Dextrinen kann auf die WO 03/087164 verwiesen werden.

Bezüglich der Herstellung von Eisen(III)-Pullulan-Komplexverbindungen kann auf die WO 02/46241 verwiesen werden.

Die erfindungsgemäß verwendeten Eisen(III)-hydroxid-Komplexverbindungen werden bevorzugt oral verabreicht. Prinzipiell können sie aber auch parenteral, wie intravenös, aber auch intramuskulär verabreicht werden. Die orale tägliche Dosis beträgt beispielsweise zwischen 1 0 und 500 mg Eisen/Tag der Anwendung. Die Verabreichung kann bedenkenlos über einen Zeitraum von mehreren Monaten bis zur Verbesserung des Eisenstatus, reflektiert durch den Hämoglobin-Wert, die Transferrin-Sättigung und den Ferritin-Wert, der Patienten eingenommen werden. Die orale Verabreichung erfolgt bevorzugt in Form einer Tablette, einer Kapsel, einer wässrigen Lösung oder Emulsion, als Granulat, Kapsel, Gel oder als Sachet. Die Anwendung von Lösungen oder Emulsionen ist besonders bei Kindern in der Form von Sirups bzw. Säften, Tropfen etc. bevorzugt. Dazu können die Eisen(III)-hydroxid-Dextrin-KomplexVerbindungen mit üblichen pharmazeutischen Träger- bzw. Hilfsstoffen in die geeignete Verabreichungsform gebracht werden. Dazu können übliche Bindemittel bzw. Gleitmittel, Verdünnungsmittel, Desintegrationsmittel etc. verwendet werden.

Die erfindungsgemäße Verwendung kann bei Kindern, Jugendlichen und Erwachsenen erfolgen, welche an chronisch entzündlichen Darmerkrankungen leiden, bevorzugt bei Erwachsenen.

Die erfindungsgemäße Verwendung verläuft insbesondere mittels Verbesserung der Eisen-, Hämoglobin-, Ferritin- und Transferrinwerte, wobei die klinischen Erkrankungsaktivitätsindizes, der Darmzustand, Bauchschmerzen und Übelkeit durch die erfindungsgemäße Behandlung nicht veschlechtert werden.

### Kurze Beschreibung der Figur

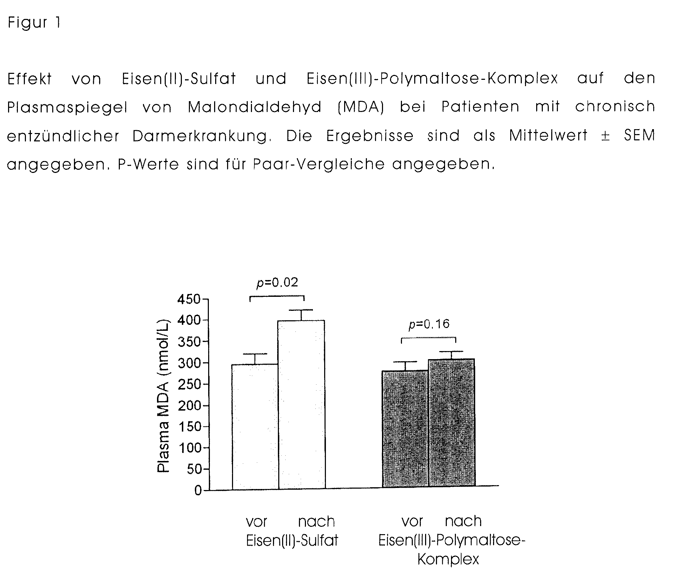

Figur 1 ist ein Diagramm, das die im Beispiel gemessenen Plasma-MDA-Spiegel vor und nach der Behandlung mit Eisen(II)-sulfat bzw. Eisen(III)-Polymaltose-Komplex zeigt.

Die Erfindung wird in ihrer Wirkungsweise durch das folgende Beispiel erläutert und belegt.

### BEISPIEL

### Patienten

41 Patienten mit chronisch entzündlicher Darmerkrankung (Colitis ulcerosa oder Morbus Crohn im aktiven oder ruhenden Zustand) und Eisenmangel (definiert durch das mittlere corpusculäre Volumen (MCV) <80 fl oder S-Ferritin < 15 µg/l oder S-löslicher Transferrin-Rezeptor > 1.54 mg/l) wurden nach dem Zufallsprinzip in zwei Gruppen aufgeteilt. Patienten, die während 6 Wochen vor Durchführung der Studie eine Eisentherapie oder Bluttransfusionen, eine weniger als zwei Monate vor Beginn der Studie beginnende Azathioprin-Behandlung oder eine Infliximab-Behandlung erhalten hatten, an Cobalamin- oder Folsäuremangel, Krebs oder Nierenerkrankungen litten oder schwanger waren, wurden ausgeschlossen. Die Untersuchung von Blut, Urin und Stuhl sowie die klinische Beurteilung der Erkrankung erfolgten am Tag 1 und 15.

### Medikation

Die Behandlung erfolgte in Gruppe 1 mit Eisen(II)-sulfat (Nycoplus Ferro-Retard®, Nycomed Pharma AS, Norwegen), mit einer Tablette (100 mg) (entspr. 100 mg Fe²⁺) morgens und einer Tablette (100 mg) abends zwischen den Mahlzeiten während 14 Tagen und in Gruppe 2 mit Eisen(III)-Polymaltose-Komplex (Maltofer Filmtabletten®, Vifor International AG, Schweiz) mit zwei Tabletten (insges, 200 mg) (entspr. 200 mg Fe(III)) einmal täglich morgens während der Mahlzeit während 14 Tagen. Die Einnahme erfolgte nach den Herstellerempfehlungen. Die Patienten-Compliance wurde definiert als Verbrauch der ausgegebenen Tabletten, wobei 80% als zufriedenstellend betrachtet wurde.

### Laboruntersuchungen

Blutproben wurden nach Fasten während der Nacht am Morgen von Tag 1 und Tag 15 entnommen.

Plasma-Malondialdehyd (MDA), Plasmaaminothiophenole, Plasma-Vitamine A, E und C und Plasma-Betacarotin wurden durch Hochleistungsflüssigkeitschromatographie (HPLC) bestimmt wie in der Literatur beschrieben (Svardal, AM., Manssor, MA., Ueland, PM., Anal. Biochem. 1990; 184:338-346; Vaagenes, H., Muna, ZA., Madsen, L., Berge, RK., Lipids 1998; 33:1131-1 137).

Routine-Laboruntersuchungen umfassten die Bestimmung von Blut-Hämoglobin, die Blut-Reticulocytenzählung, die Bestimmung des mittleren corpusculären Volumens (MCV), des mittleren corpusculären Hämoglobins (MCH), der mittleren corpusculären Hämoglobin-Konzentration (MCHC), eine Blut-Erythrocytenzählung, Blut-Leukocytenzählung, und Blut-Plättchenzählung, die Bestimmung des Reticulocyten-Hämoglobins (CHr), die Zählung der hypochromen roten Zellen (HYPO), die Bestimmung von Serum-Ferritin und Serum-Eisen, die Bestimmung der Serum-Eisen-Gesamtbindungskapazität, des Serum-löslichen Transferrin-Rezeptors, des Serum-C-reaktiven Proteins (S-CRP), die Messung der Blut-Erythrocyten-Sedimentationsgeschwindigkeit (B-ESR),die Bestimmung von Serum-Protein und Serum-Albumin.

Urinproben wurden am Morgen von Tag 1 und Tag 15 genommen und auf Kreatinin untersucht. Butyl-hydroxy-toluol (BHT) wurde zu 2 ml Urin gegeben auf eine Endkonzentration von 20 mM. Die Proben wurden dann bis zur Analyse von Urin-8-isoprostaglandin F_{2α} (8-Iso-PG F_{2α}) bei -80°C gelagert, Die Analyse erfolgte durch Gaschromatographie-Massenspektrometrie nach der Methode von Nourooz-Zadeh et al. (Nourooz-Zadeh J., Gopaul NK., Barrow S., Mallet Al., Anggard EE., J. Chromatogr. B. Biomed, Appl. 1995; 667:199-208), wurde aber hinsichtlich der Urinmatrix durch Weglassen des anfänglichen Hydrolyseschritts und Anwendung des Festphasenprotokolls von Lee et al. (Lee CY, Jenner AM., Halliwell B" Biochem. Biophys. Res. Commun. 2004; 320: 696-702) modifiziert.

### Klinische Erkrankungsaktivität

Der Zustand der klinischen Erkrankung wurde vor (Tag 1) und nach (Tag 15) der Eisentherapie aufgenommen. Die klinische Erkrankungsaktivität wurde bei Patienten mit Morbus Crohn mit dem "Harvey-Bradshaw Simple Index of Crohn's Disease Ativity" (Harvey, RF., Bradshaw, JM., Lancet, 1 980; 1:514) bewertet. Der Harvey-Bradshaw Simple Index basiert auf 5 Parametern: allgemeines Wohlbefinden, Bauchschmerzen, Stuhlhäufigkeit, Abdominalmasse und extraintestinale Komplikationen. Der Maximalwert ist 25 und Werte von ≥5 zeigen aktiven Morbus Crohn an.

Bei Patienten mit Colitis ulcerosa wurde der "Simple Clinical Colitis Activity Index" aufgezeichnet (Walmsley, RS., Ayres, RC., Pounder, RE., Allan, RN., Gut 1998; 43; 29-32), Der Simple Clinical Colitis Activity Index basiert auf 6 Parametern: allgemeines Wohlbefinden, Stuhlhäufigkeit tags und nachts, Stuhldrang, Blut im Stuhl und extraintestinale Komplikationen. Der Maximalwert ist 20 und Werte von ≥ 4 zeigen aktive Colitis ulcerosa an.

Der Harvey-Bradshaw Simple Index und der Simple Clinical Colitis Activity Index sind gleich im Hinblick auf den Aufbau und die klinische Bedeutung einer gegebenen Änderung der Werte. Um die gemeinsame Berücksichtigung der Ergebnisse von Patienten mit Morbus Crohn und Colitis ulcerosa zu ermöglichen, wurden die Aktivitätswerte als tatsächlicher Wert dividiert durch den Maximalwert berechnet.

Alle Patienten führten die jeweilige Crohn Disease Activity Index (CDAI) Tagebuchkarte (Best, WR., Becktel, JM., Singleton, JW., Kern, F. Jr., Gastroenterology 1976; 70:439-444) in der Woche vor Beginn der Eisentherapie und während der zweiwöchigen Eisentherapie. Die CDAI-Tagebuchkarte beinhaltet die tägliche Aufzeichnung des allgemeinen Wohlbefindens, der Bauchschmerzen und der Anzahl der flüssigen oder sehr weichen Stühle. Die Summe von sieben täglichen Aufzeichnungen ergibt einen Wert für jedes Symptom. Je höher der Wert, desto mehr ist der Patient beeinträchtigt. Die Arzneimittelgabe während der Studie erfolgte während 14 Tagen und daher wird der Mittelwert für die zwei Wochen für die Analyse verwendet. Die Patienten dokumentierten auch das Auftreten von Übelkeit vor und während der Eisentherapie.

Patienten, die die Arzneimittelbehandlung wegen einer Verschlechterung der Symptome abbrachen, wurden in die Analyse der klinischen Erkankungsaktivität und der Symptomwerte einbezogen. Ihre Erkrankungsaktivitätswerte wurden um zwei Punkte erhöht, und die Symptomwerte wurden um einen Punkt pro Tag erhöht.

### Ziel und Ergebnisse

Das primäre Ziel der Studie war ein Vergleich der Wirkung von oralem Eisen(II)-sulfat und oralem Eisen(III)-Polymaltose-Komplex auf Marker für oxidative Gewebeschäden, Die primären Ergebnisse waren Plasma-MDA und Urin-iso-PGF_{2α}. Das zweite Ziel war der Vergleich der Wirkung der beiden Eisenformulierungen auf die klinische Erkrankungsaktivität und spezifische Symptome. Die Behandlungszeit war zu kurz für eine Studie der klinischen Wirksamkeit auf die Behebung des Eisenmangels.

### Statistische Analyse

Die Differenzen innerhalb der und zwischen den Gruppen wurden mit dem gepaarten und ungepaarten Student t-Test beurteilt, und der Mittelwert der Differenzen und das 95%-Konfidenzintervoll sind angegeben. Die Werte wurden unter Verwendung des Wilcoxon-Test für Paar-Differenzen analysiert, und es sind der Median und der Bereich angegeben. Der Vergleich von Verhältnissen wurde mit dem Fisher-Exakt-Test beurteilt. P-Werte von weniger als 0,05 werden als statistisch signifikant betrachtet. Die Daten wurden unter Verwendung des Statistik-Softwarepakets GraphPad Prism 4 for Windows (GraphPad Software, Inc., San Diego, USA) analysiert.

### Ergebnisse

41 Patienten (Tabelle I) wurden zur Behandlung entweder mit Eisen(II)-sulfat (n=21) oder Eisen(III)-Polymaltose-Komplex (n=20) nach dem Zufallsprinzip aufgeteilt. 37 Patienten durchliefen den Versuch protokollgemäß. Bei diesen Patienten ergab die Zahlung der Tabletten eine vergleichbare Compliance bei den mit Eisen(II)-sulfat (100% (82-100)) und mit Eisen(III)-Polymaltose-Komplex (100% (86-100)) behandelten Patienten. Drei Patienten (1 Morbus Crohn, 2 Colitis ulcerosa) brachen die Einnahme von Eisen(II)-sulfat nach 1 , 4 bzw. 5 Tagen ab, und ein Patient (Morbus Crohn) brach die Behandlung mit Eisen(III)-Polymaltose-Komplex nach 1 Tag ab. Sie erlitten alle nicht tolerierbare Darmbewegungen, Bauchschmerzen und Übelkeit. Diese Patienten wurden von der Analyse der Laborwerte ausgeschlossen, sind in der Analyse der klinischen Erkrankungsaktivität und der Symptomwerte aber eingeschlossen.

### Marker für oxidativen Stress

Die Behandlung mit Eisen(II)-sulfat erhöhte die Plasma-MDA-Werte deutlich um 95 nmol/l (Cl 18 bis 1 71 ; p=0,01 8) (Figur 1) und erhöhte die Urin-Iso-PGF_{2α} -Werte um 194 pg/mg Kreatinin (Cl-58 bis 447; p =0,12). Die Behandlung mit Eisen(III)-Polymaltose-Komplex veränderte Plasma-MDA (p=0,16) (Figur 1) oder Urin-Iso-PGF_{2α} (p=0,56) nicht signifikant (Tabelle II). Plasma-Vitamine A, C und E, Beta-Carotin, Glutathion, Cystein, CysteinylGlycin und Homocystein waren nach beiden Behandlungen unverändert (Tabelle II). Beim Vergleich der Behandlung von Eisen(II)-sulfat und Eisen(III)-Polymaltose-Komplex unterschieden sich die Veränderungen (vorhernachher) in Plasma-MDA (p=0,08) und Urin-Iso-PGF_{2α} (p=0,28) nicht signifikant. Die mittleren Plasma-MDA-Werte der beiden Gruppen waren aber nach der jeweiligen Behandlung signifikant unterschiedlich (p=0,007), wobei in der Eisen(II)-sulfat-Gruppe höhere MDA-Werte auftraten (Tabelle II). Keiner der Urin-oder Plasma-Parameter korrelierte mit den klinischen Aktivitäts-Indizes.

### Klinische Erkrankungsaktivität und Symptome

Die Wertzahlen der klinischen Erkrankungsaktivität sind in Tabelle III angegeben. Weder die Behandlung mit Eisen(II)-sulfat (p=0,45) noch die Behandlung mit Eisen(III)-Polymaltosekomplex (p=0,80) veränderte die klinischen Erkrankungsaktivitäts-Indizes wesentlich, und die Veränderungen zwischen den Behandlungen unterschieden sich nicht (p=0,81), wobei sich während der Behandlung mit Eisen(II)-sulfat die Anzahl der Stuhlgänge (von 19 (7-106) auf 24 (7-55); p=0,0087) erhöhte, Eisen(III)-Polymaltosekomplex die Gesamtzahl der Stuhlgänge pro Woche jedoch nicht veränderte (von 17(7-46) auf 17 (6-66); p=0,25, Weder Eisen(II)-sulfat noch Eisen(III)-Polymaltose-Komplex hatte Einfluss auf das allgemeine Wohlbefinden oder auf die Bauchschmerzen-Wertzahl (Daten nicht angegeben), Vermehrte Übelkeit wurde von 9/21 Patienten bei Eisen(II)-sulfat und bei 7/20 Patienten beim Eisen(III)-Polymaltosekomplex angegeben (p=0,75).

### Routine-Laboruntersuchungen

Die Routine-Laboruntersuchungen sind in Tabelle III angegeben. Weder Eisen(II)-sulfat noch Eisen(III)-Polymaltosekomplex erhöhte Blut-Hämoglobin, Nur Eisen(II)-sulfat hatte einen signifikanten Einfluss auf die biochemischen Marker von Eisenmangel, mit einem Anstieg in Reticulocyten-Hämoglobin (1,9 pg mi Cl 0,01 bis 3,8; p=0,049), S-Ferritin (12 µg/l mit Cl 6 bis 17; p=0,0003) und Blut-Reticulozytenzahl (0,016 x10¹²/l mit Cl -0,004 bis 0,036; p=0,1 0), und einer Abnahme der hypochromen roten Zellen (-2,5% mit Cl - 4,6 bis -0,3; p=0,026), des Serum-löslichen Transferrin-Rezeptors (-0,21 mg/l mit Cl -0,31 bis -0,11; p=0,0005) und der Serum-Gesamteisenbindungskapazität (-7 µmol/l mit Cl -10 bis -4; p<0,0001), Eisen(III)-Polymaltose-Komplex erhöhte nur die Blut-Reticulozytenzahl (0,016x10¹²/l mit Cl ,001 bis 0,030; p=0,034).

Aus den Ergebnissen der Studie wird deutlich, dass bei Patienten mit chronisch entzündlichen Darmerkrankungen eine gute Verträglichkeit der Eisentherapie mit Eisen(III)-Polymaltosekomplex erreicht wird, wobei insbesondere die Stuhlfrequenz gegenüber Eisen(II)-sulfat erniedrigt war und weniger Patienten die Studie wegen Darmbeschwerden abbrachen. Darüber hinaus ist der oxidative Stress bei der erfindungsgemäßen Therapie deutlich geringer als mit Eisen(II)-sulfat.

**Tabelle I, Patientencharakteristik. Median (Bereich) für Alter, Anzahl und weitere Parameter**

| | Eisen(II)-sulfat | Eisen(III)-Polymaltose-Komplex |
|---|---|---|
| M. Crohn/Colitis ulcerosa | 13/8 | 11/9 |
| Weiblich/männlich | 13/8 | 12/8 |
| Alter | 41 (17-69) | 31.5 (16-68) |
| Krankheitslokalisierung | | |
| M. Crohn* terminales Ileum | 2 | 2 |
| Colon | 4 | 1 |
| Ileocolon | 3 | 4 |
| oberer Gl | 4 | 4 |
| Krankheitslokalisierung UC | 1 | 2 |
| distale Colitis | 3 | 3 |
| subtotale Colitis | 4 | 4 |
| Gesamt-Colitis | | |
| Gleichzeitige | | |
| Medikation | | |
| 5-ASA | 13 | 1 |
| Sulphasalazin | 1 | 2 |
| Steroide | 7 | 5 |
| Azathioprin | 6 | 5 |
| Keine | 1 | 5 |

| | | |
|---|---|---|
| *Krankheitslokalisierung für M. Crohn wie von der Wiener Klassifikation für M. Crohn definiert. UC: Colitis ulcerosa | | |

**Tabelle II. Marker für oxidativen Stress, Mittelwert (SEM).**

| | Eisen(II)-sulfat | | Eisen(III)-Polymaltose-Komplex | |
|---|---|---|---|---|
| Parameter | Vorher | Nachher | Vorher | Nachher |
| U-8-iso-PGF_{2α} (pg/mg Kreatinin) | 417 (46) | 629 (124) | 396 (46) | 434 (64) |
| P-Malondialdehyd (nmol/L) | 294 (25) | 395 (25) * | 275 (21) | 300 (19) |
| P-Vitamin A (µmol/L) | 1 ,7 (0.2) | 1.8 (0.2) | 1.6 (0.1) | 1.9 (0,3) |
| P-Vitamin C (µmol/L) | 60,9 (6.0) | 58.6 (5.4) | 61.3 (5.1) | 54,5 (5.5) |
| P-Vitamin E (µmol/L) | 30.2 (1.8) | 29.3 (1.5) | 29.3 (1.6) | 28.3 (1.7) |
| P-beta-Carotin (µmol/L) | 0,67 (0.09) | 0.67 (0.10) | 0,59 (0.13) | 0,57 (0,09) |
| P-Glutathion (µmol/L) | 5.05 (0.48) | 5.08 (0.54) | 5.22 (0.30) | 5.43 (0.43) |
| P-Cystein (µmol/L) | 203 (11) | 199 (13) | 211 (11) | 209 (11) |
| P-Cysteinylglycin (µmol/L) | 16.7 (1.1) | 16.6 (1.2) | 18.7 (0.9) | 18.5 (1.1) |
| P-Homocystein (µmol/L) | 4.87 (0.59) | 4.58 (0.47) | 6.53 (1.16) | 6.04 (0,94) |

| | | | | |
|---|---|---|---|---|
| * Signifikant verschieden vom Spiegel vor der Behandlung (p<0.05). Daten von 4 Patienten, die die Behandlung abbrachen, sind nicht in der Tabelle enthalten. P: Plasma; U: Urin | | | | |

**Tabelle III. Routine-Laboruntersuchungen. Mittelwert (SEM)**

| | | Eisen(II)-sulfat | | Eisen(III)-Polymaltose-Komplex | |
|---|---|---|---|---|---|
| Parameter | Normal | Vorher | Nachher | vorher | nachher |
| B-Hämoglobin (g/dL) | w 11.6-16.0 | 13.1 (0.4) | 13.3 (0.3) | 12.5 (0.3) | 12.5 (0.3) |
| | m 13.2-16.6 | | | | |
| B-Hämatokrit (%) | w 36-46 | 41 (1) | 42 (1)* | 39 (1) | 40 (1) |
| | m 37-49 | | | | |
| MCV (fL) | 80-102 | 86 (1.6) | 87 (1.3)* | 84 (1.8) | 85(1.6) |
| MCH (pg) | 27-35 | 27 (0.8) | 28 (0.8)*# | 27 (0.7) | 27 (0.7) |
| MCHC (g/dL) | 31.0-36.0 | 31.8 (0.4) | 32.0 (0.3) | 31.6 (0.3) | 31.2 (0.3) |
| Reticulocyten-Hämoglobin (CHr) (pg) | >28 | 29.3 (0.8) | 31.1(0.7)* | 29.1 (0.8) | 29.5 (0.7) |
| Hypochrome rote Zellen (HYPO) (%) | <5 | 10.4 (3.6) | 8.8 (3.2)* | 10.3 (3.0) | 10.6 (2.8) |
| B-Erythrocytenzahl (10¹²/L) | w 3.7-5.5 | 4.8 (0.1) | 4.8 (0.1) | 4.7 (0.1) | 4.7 (0,1) |
| | m 4.0-5.8 | | | | |
| B-Reticulocytenzahl (10¹²/L) | 0.030-0.100 | 0.068 (0.006) | 0.084 (0.007) | 0.059 (0.006) | 0.075 (0.008)* |
| B-Leucocytenzahl (10⁹/L) | 3.5-11.0 | 6.5 (0.5) | 6.3 (0.4) | 6.9 (0.6) | 7.0 (0.7) |
| B-Plättchenzahl (10⁹/L) | 140-400 | 324 (23) | 306 (21) | 347 (18) | 343 (21) |
| S-Gesamteisenbindungs-kapazität (µmol/L) | 49-85 | 81 (2) | 74 (2)*# | 77 (2) | 77 (2) |
| S-Eisen (µmol/L) | 9.0-33.0 | 11.1 (2.0) | 14.2 (2.2) | 8.8 (0.8) | 8.9 (1.5) |
| S-Ferritin (µg/L) | w 15-160 | 13 (2) | 25 (3)*# | 13 (2) | 13 2 |
| | m 25-200 | | | | |
| S-löslicher Transferrin-Rezeptor (mg/L) | 0.84-1.54 | 1.95 (0.18) | 1.77 (0.13)* | 2.08 (0.24) | 2.03 (0.21) |
| B-ESR (mm/h) | w <20 | 11 (2) | 10 (2) | 22 (3) | 20 (3)* |
| | m <15 | | | | |
| S-CRP (mg/L) | <10 | 7 (2) | 6 (2) | 12 (3) | 11 (2) |

| | | | | | |
|---|---|---|---|---|---|
| * Signifikant verschieden vom Spiegel vor der Behandlung (p<0.05). # Signifikant unterschiedliche Änderung verglichen mit Eisen(III)-Polmaltose-Komplexp<0,05), Daten von vier Patienten, die die Behandlung abbrachen, sind nicht in der Tabelle enthalten w: weiblich m: männlich B: Blut S: Serum | | | | | |

## Patentansprüche

1. Verwendung von Eisen(III)-Komplexverbindungen mit Kohlenhydraten oder Derivaten davon zur Herstellung eines Arzneimittels zur Behandlung von Eisenmangel-Zuständen bei Patienten mit chronisch entzündlicher Darmerkrankung.

2. Verwendung nach Anspruch 1 , worin die Kohlenhydrate aus der Gruppe ausgewählt werden, die aus Dextranen und Derivaten davon, Dextrinen und Derivaten davon sowie Pullulan, Oligomeren und/oder Derivaten davon besteht.

3. Verwendung nach Anspruch 1 oder 2, worin die Kohlenhydrate aus oxidierten oder hydrierten Dextrinen ausgewählt werden.

4. Verwendung nach Anspruch 1 oder 2, worin die Eisen(III)-Komplexverbindung eine Eisen(III)-Polymaltose-Komplexverbindung ist.

5. Verwendung nach Anspruch 4, worin die Eisen(III)-Polymaltose-Komplexverbindung ein Molekulargewicht im Bereich von 20000 bis 500000 Dalton besitzt.

6. Verwendung nach einem der Ansprüche 1 bis 5, worin die Eisen(III)-Komplexverbindung eine Eisen(III)-Komplexverbindung mit einem Oxidationsprodukt von einem oder mehreren Maltodextrinen ist.

7. Verwendung nach Anspruch 6, worin die Eisen(III)-Komplexverbindung ein wasserlöslicher Eisen-Kohlenhydrat-Komplex ist, erhältlich aus einer wässrigen Eisen(III)-Salzlösung und einer wässrigen Lösung des Produktes der Oxidation von einem oder mehreren Maltodextrinen mit einer wässrigen Hypochloritlösung bei einem pH-Wert im alkalischen Bereich, wobei beim Einsatz von einem Maltodextrin dessen Dextrose-Äquivalent bei 5 bis 37 und beim Einsatz eines Gemisches aus mehreren Maltodextrinen das Dextrose-Äquivalent des Gemisches bei 5 bis 37 und das Dextrose Äquivalent der am Gemisch beteiligten einzelnen Maltodextrine bei 2 bis 40 liegt.

8. Verwendung nach einem der Ansprüche 1 bis 7 zur Herstellung eines Arzneimittels zur oralen oder parenteralen Verabreichung.

9. Verwendung nach einem der Ansprüche 1 bis 8, worin das Arzneimittel in der Form einer Tablette, einer wässrigen Lösung oder Emulsion, als Granulat, Kapsel, Gel oder als Sachet vorliegt.

10. Verwendung nach einem der Ansprüche 1 bis 9, wobei die chronisch entzündliche Darmerkrankung Morbus Crohn und/oder Colitis ulcerosa ist,
